# EUROPEAN PATENT APPLICATION

(11) **EP 2 638 959 A1**
(43) Date of publication of application: **18.09.2013**
(21) Application number: 11839310.7
(22) Date of filing: 09.11.2011
(51) Int. Cl.: B01J 19/08, A61L 9/22, H05H 1/24

(54) **PLASMA GENERATOR, AND PLASMA GENERATING METHOD**

(30) Priority: 09.11.2010 JP 2010250924
(71) Applicant: Samsung Electronics Co., Ltd., Suwon-si, Gyeonggi-do, 443-742 (KR)
(72) Inventor: MIYAMOTO, Makoto, Yokohama-shi Kanagawa 230-0027 (JP); TAKENOSHITA, Kazutoshi, Yokohama-shi Kanagawa 230-0027 (JP); KUMAGAI, Yuki, Yokohama-shi Kanagawa 230-0027 (JP); NAKAYAMA, Yoko, Yokohama-shi Kanagawa 230-0027 (JP); NOJIMA, Hideo, Yokohama-shi Kanagawa 230-0027 (JP)
(74) Representative: Walaski, Jan Filip
(86) International application number: PCT/JP2011/075820
(87) International publication number: WO 2012/063857

(57) **Abstract**

The present invention obtains both the feature of deodorizing by means of active species and the feature of killing floating and attached bacteria by releasing the active species to the exterior of a device. The present invention is provided with a pair of electrodes (21, 22), electrode (21) being arranged with the dielectric film (21a) on the surface facing electrode (22) and electrode (22) being arranged with dielectric film (22a) on the surface facing electrode (21), wherein plasma is discharged when a predetermined voltage is applied between the electrodes (21, 22). The present invention is configured in a manner such that a fluid through-hole (21b, 22b) is disposed on and through each electrode (21, 22) on a corresponding location, and is characterized in that at least a portion of the outline of the corresponding fluid through-holes (21b, 22b) are positioned at a different position from one another when viewing from face plate direction of the electrodes.

## Description

### [Technical Field]

The present invention relates to a plasma generator and a plasma generating method.

### [Background Art]

Recently, there is an increasing need for air quality control of a living environment such as sterilization and deodorization, due to increased risk of infection such as seen in an increase in carriers of atopy, asthma, and allergic symptoms and explosive prevalence of new influenza. In addition, as living becomes rich, an amount of food storage and a chance of storing leftover food are increased. Accordingly, the importance of environmental control in storage equipment represented as a refrigerator is also growing.

In the prior art for the purpose of controlling air quality of a living environment, physical control as represented by a filter is generally used. According to the physical control, relatively large dust and debris floating in the air may be captured, and bacteria, viruses, or the like may also be captured depending on the size of a filter hole. In addition, when there are an infinite number of adsorption sites such activated carbon, it may also be possible to capture malodorous molecules. However, there are problems in that air in a space to be controlled is required to evenly pass through the filter in order to be captured, the apparatus is increased in size, and a maintenance cost such as filter replacement is also increased while it has no effect on adhesive substances. Therefore, as a means to enable sterilization and deodorization of adhesive substances, it may be exemplified to release chemically active species to a space desired to perform sterilization and deodorization. In spraying of chemicals or release of flavoring agents or deodorant, it is necessary to prepare the active species in advance and regular replenishment thereof is essential. On the other hand, a means to perform sterilization and deodorization using the chemically active species generated by generating plasma in the atmosphere is increased in recent years.

Technologies to perform sterilization and deodorization by ions and radicals (hereinafter, referred to as "active species") generated by discharge of plasma into the atmosphere may be classified into the following two types:
(1) a so-called passive type plasma generator in which bacteria and viruses floating in the atmosphere (hereinafter, referred to as "floating bacteria") or malodorous substances (hereinafter, referred to as "odor") react with active species within a limited capacity in the apparatus (for example, Patent Document 1); and
(2) a so-called active type plasma generator in which active species generated by a plasma generating portion are released into a closed space (e.g., a living room, a toilet, a car interior, or the like) having a larger capacity than (1) released into, and the active species in the atmosphere react with floating bacteria and odor by a collision therewith (for example, Patent Document 2).

The passive type plasma generator of (1) has an advantage that high sterilization and deodorization effects may be expected because active species of high concentration are generated by generation of plasma in the small capacity. On the other hand, the apparatus has a disadvantage that the size thereof is increased because floating bacteria and odor are required to be introduced into the apparatus, and a filter for adsorption or decomposition is required to be separately installed in order to prevent ozone from leaking out of the apparatus since the ozone is likely to occur as a by-product from plasma generation.

Next, the active type plasma generator of (2) has an advantage that the apparatus may be relatively small, and sterilization of bacteria adhered to a surface of clothing (hereinafter, referred to as "adhesive bacteria") and decomposition of odor adsorbed onto the surface may be expected in addition to sterilization of floating bacteria and decomposition of odor in the air. On the other hand, the apparatus has a disadvantage that only long-lived active species cannot help but expect sterilization and deodorization effects because active species are diffused within the closed space, which is very large compared to the volume of the apparatus, and have low concentration. As a result, the deodorization effect may not be nearly expected in a space having high odor concentration (high concentration 10,000 times the concentration of active species).

From the above, in the passive type plasma generator, the effect is limited only to floating bacteria and odor contained in an air stream flowing into the apparatus. On the other hand, in the active type plasma generator, the effect cannot help but be expected only with respect to floating bacteria, adhesive bacteria, and odor having low concentration. In other words, only either "sterilization and deodorization of floating bacteria" or "sterilization of floating bacteria and adhesive bacteria having low concentration and deodorization of adhesive odor" may be realized using the prior art.

However, there are some situations where sterilization of adhesive bacteria having high concentration and deodorization of odor having high concentration are required to be simultaneously performed in a daily life environment. The most typical example is a refrigerating chamber of a refrigerator in which many bacteria adhered to surfaces of food and a storage container surfaces exist and odor arising from food itself and decayed leftover food also exists.

### [Citation List]

### [Patent Document]

Patent Document 1: Japanese Unexamined Patent Application Publication No. 2002-224211
Patent Document 2: Japanese Unexamined Patent Application Publication No. 2003-79714

### [Disclosure]

### [Technical Problem]

Therefore, the present invention is a technique to simultaneously realize both sterilization and deodorization of adhesive bacteria, and it is a main object of the present invention to increase a generation amount of active species, so as to simultaneously include both a passive function which deodorizes adhesive bacteria using active species by generation of plasma and an active function which releases the active species outside an apparatus to sterilize the adhesive bacteria.

### [Technical Solution]

In accordance with an aspect of the present invention, a plasma generator includes a pair of electrodes, in a case where a predetermined voltage is applied between the electrodes to discharge plasma, fluid circulation holes being respectively provided at corresponding positions of each electrode and passing through the electrode, at least a portion of an outline of each corresponding fluid circulation hole being arranged at positions different from each other when viewed from a face plate direction of the electrode. Here, the corresponding positions mean that the fluid circulation holes formed in the pair of electrodes are substantially in the same positions and face each other when viewed from a face plate direction of each electrode. In addition, the corresponding positions mean the same substantially coordinate position (x, y) at both electrodes when viewing the pair of electrodes on the x-y plane from the z-axis direction in the orthogonal coordinate system.

In accordance with such a configuration, since at least a portion of an outline of each corresponding fluid circulation hole is arranged at positions different from each other, it may be possible to increase a contact area between fluid passing through the fluid circulation hole and the plasma. Thus, it is possible to increase a generation amount of active species such as ions or radicals and to sufficiently realize a deodorization function by the active species and a function which releases the active species outside an apparatus to sterilize floating bacteria and adhesive bacteria.
Here, at least one side of the pair of electrodes is provided with a dielectric film, and thus a spacer to define a gap for plasma formation between the respective electrodes 21 and 22 is not required, and the gap may be defined between the facing surfaces.

As an aspect which is specifically realized so that at least a portion of an outline of each corresponding fluid circulation hole is arranged at positions different from each other, a size of the fluid circulation hole formed in the electrode of one side of the pair of electrodes may be formed to be smaller than a size of the fluid circulation hole formed in the electrode of the other side by 10 µm or more. Otherwise, the fluid circulation holes having the same opening size may also be arranged to be deviated from the opening center thereof.

In order to maximally increase a contact area between fluid and plasma in a case where at least a portion of an outline of each corresponding fluid circulation hole is arranged at positions different from each other, each fluid circulation hole may have a circular shape, and the fluid circulation hole formed in the electrode of one side and the fluid circulation hole formed in the electrode of the other side may be arranged in a concentric circular shape.

When the corresponding fluid circulation holes of the pair of electrodes are provided in plural number, it may be possible to increase a deodorization function for the active species and a sterilization function for floating bacteria and adhesive bacteria.

In order to suppress generated ozone concentration while increasing the number of active species included in fluid passing through the fluid circulation holes, a total opening area of the fluid circulation holes formed in each electrode may be within a range of 2% to 90% with respect to a total area of each electrode.

In order to increase deodorization of fluid passing through the fluid circulation hole or the passing fluid, and sterilization of floating bacteria included in the fluid or an amount of the released active species, a through hole may be provided separately from the fluid circulation holes in the electrode of one side and the through hole is blocked, at an opening of a facing surface thereof, by the electrode of the other side. Thereby, the fluid after passing through the fluid circulation hole is introduced into the through hole to come into contact with plasma, or the fluid before passing through the fluid circulation hole is introduced into the through hole to come into contact with plasma, thereby the present invention may be effective.

As an aspect of specific embodiment of the through hole, an opening size of the through hole may be formed to be smaller than an opening size of the fluid circulation hole by 10 µm or more.

The surface roughness of the dielectric film may be 0.1 µm to 100 µm. Thus, even when the pair of electrodes are laminated without using a spacer, it may be possible to form a generation space of the plasma by the surface roughness.

In order to promote the generation of active species by efficient passing of fluid through the fluid circulation hole and increase a deodorization effect, the plasma generator may include a blower mechanism to forcibly blow wind toward the fluid circulation holes.

The blower mechanism may allow a flow rate of the wind passing through the fluid circulation holes to be within a range of 0.1 m/s to 10 m/s.

A plasma generator to realize both sterilization and deodorization of the floating bacteria according to another aspect of the present invention includes a pair of electrodes, in a case where a predetermined voltage is applied between the electrodes to discharge plasma, fluid circulation holes being respectively provided at corresponding positions of each electrode and passing through the electrode, a through hole being provided separately from the fluid circulation holes in the electrode of one side and the through hole being blocked, at an opening of a facing surface thereof, by the electrode of the other side.

In accordance with such a configuration, the fluid passing through the fluid circulation hole may come into contact with plasma through the through hole, or the fluid before passing through the fluid circulation hole may come into contact with plasma through the through hole. Therefore, it is possible to increase a generation amount of active species such as ions or radicals and to sufficiently realize a deodorization function by the active species and a function which releases the active species outside an apparatus to sterilize floating bacteria and adhesive bacteria.

In order to suppress generated ozone concentration while increasing the number of active species included in fluid passing through the fluid circulation holes, a voltage applied to each electrode may be formed in a pulse shape, a peak value thereof may be set within a range of 100 V to 5000 V, and a pulse width may be set within a range of 0.1 µs to 300 µs.

In addition, a refrigerator corresponding to CFC elimination uses inflammable gas as refrigerant, there is a problem in that the plasma generators are applied to the refrigerator used with inflammable gas. Thus, the plasma generator may include an explosion-proof mechanism, wherein the explosion-proof mechanism has protective covers disposed to the outer sides of the pair of electrodes, and is configured so that flame generated by plasma through introduction of inflammable gas into the fluid circulation holes is not spread beyond the protective covers to the outside.

In order to secure safety of the plasma generator, the protective covers may have metal meshes disposed at the outer sides of the pair of electrodes, a wire diameter of each metal mesh is within a range of 1.5 mm or less, and an opening ratio of the metal mesh is 30% or more.

In accordance with a further aspect of the present invention, a plasma generating method using a pair of electrodes is provided, wherein fluid circulation holes are respectively provided at corresponding positions of each electrode and pass through the electrode, at least a portion of an outline of each corresponding fluid circulation hole is arranged at positions different from each other when viewed from a face plate direction of the electrode, so that a predetermined voltage is applied between the electrodes to discharge plasma.

### [Advantageous Effects]

In accordance with the present invention having such a configuration, it may be possible to simultaneously realize a deodorization function by active species and a function which releases the active species outside the apparatus to sterilize floating bacteria and adhesive bacteria.

### [Description of Drawings]

The above and other objects, features and other advantages of the present invention will be more clearly understood from the following detailed description taken in conjunction with the accompanying drawings, in which:
FIG. 1 is a view illustrating a plasma generator according to an embodiment of the present invention;
FIG. 2 is a diagram illustrating an operation of the plasma generator;
FIG. 3 is a top view illustrating an electrode portion;
FIG. 4 is a cross-sectional view illustrating an electrode portion and an explosion-proof mechanism;
FIG. 5 is an enlarged cross-sectional view illustrating a configuration of a facing surface of the electrode portion;
FIG. 6 is a partial enlarged top view and a cross-sectional view schematically illustrating a fluid circulation hole and through hole;
FIG. 7 is a graph illustrating opening ratio dependence of ion number density and ozone concentration;
FIG. 8 is a graph illustrating ion number density per unit peripheral length (a ratio to a symmetrical form) by an electrode shape;

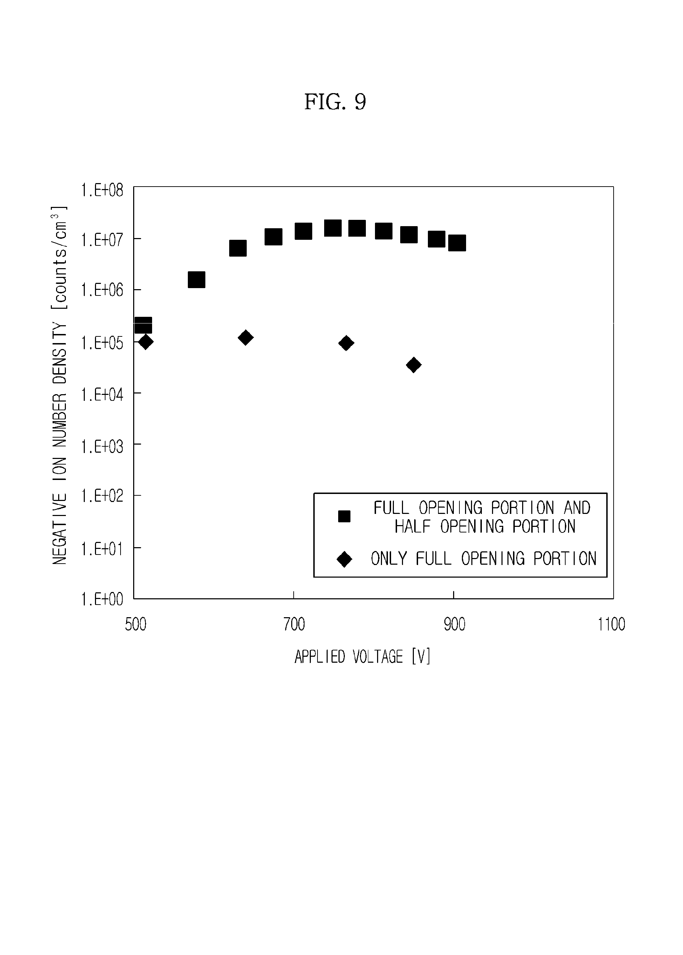

FIG. 9 is a graph illustrating an increase in ion number density by a change in electrode structure;
FIG. 10 is a graph illustrating a difference in sterilization effect by an electrode shape;
FIG. 11 is a graph illustrating a difference in deodorization effect by an electrode shape;
FIG. 12 is a view schematically illustrating plasma generation and a deodorization reaction field;
FIG. 13 is a view schematically illustrating sterilization of adhesive bacteria by released active species;

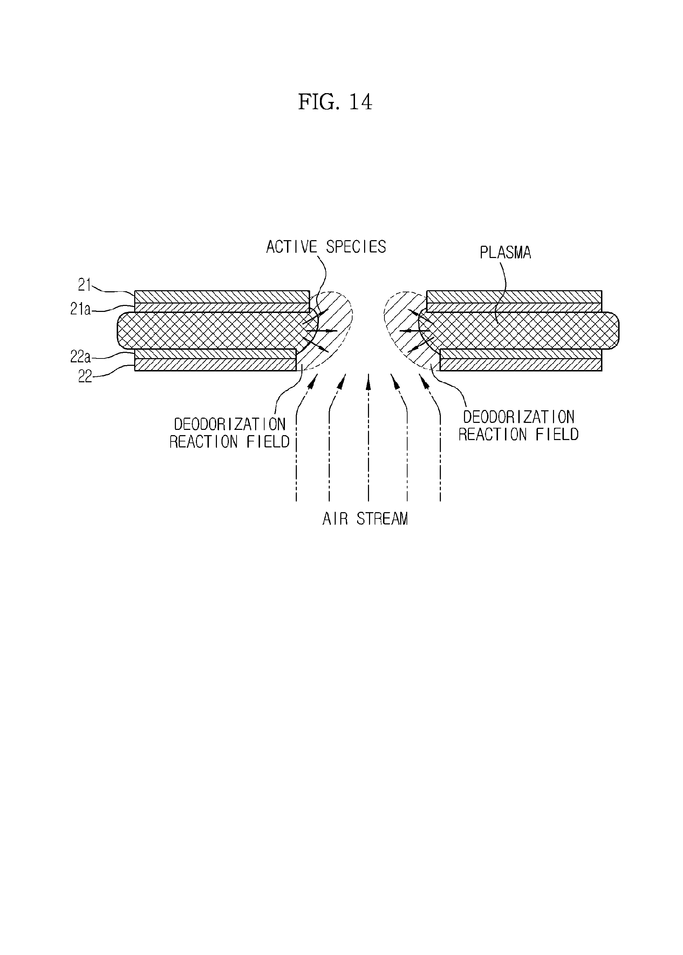

FIG. 14 is a view schematically illustrating an improvement in deodorization efficiency by the fluid circulation hole of the present embodiment;
FIG. 15 is a view schematically illustrating release of active species by the fluid circulation hole of the present embodiment;

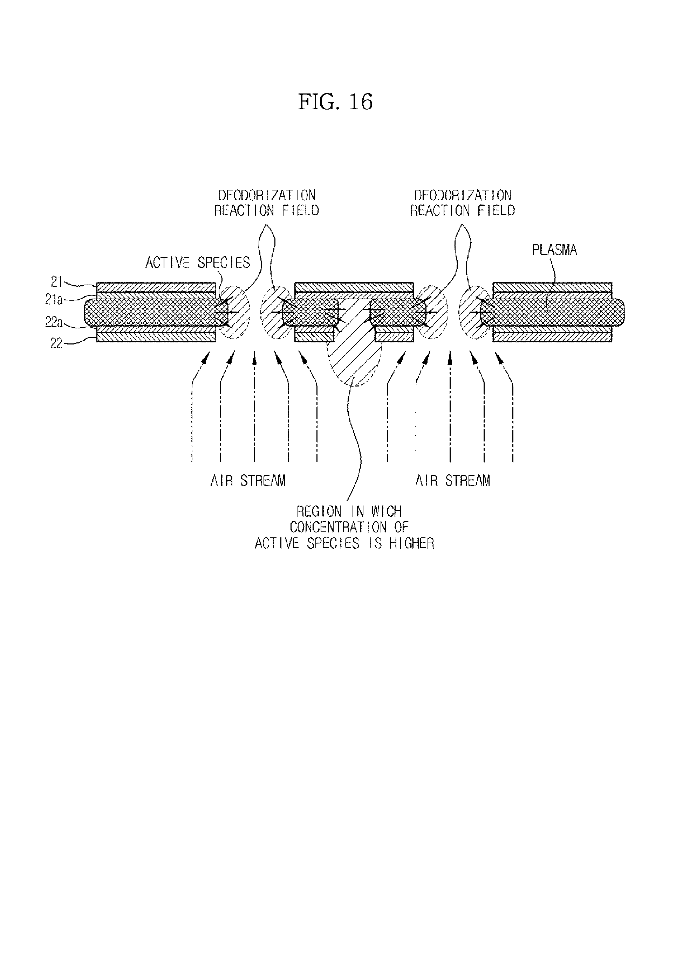

FIG. 16 is a view schematically illustrating an improvement in deodorization efficiency by an active species region having high concentration of the through hole of the present embodiment;
FIG. 17 is a view schematically illustrating release of active species having high concentration in the through hole of the present embodiment;
FIG. 18 is a view schematically illustrating ignition by plasma at the time of abnormality and prevention of flame spread by the explosion-proof mechanism; and
FIG. 19 is a graph illustrating a parameter region of a metal mesh meeting explosion-proof ability and ion releasing efficiency.

### [Reference Signs List]

- 100:: a plasma generator
- 21:: an electrode of one side
- 22:: an electrode of the other side
- 21a, 22a:: dielectric films
- 21b, 22b:: fluid circulation holes
- 21c:: a through hole
- 3:: a blower mechanism
- 4:: an explosion-proof mechanism
- 41:: a protective cover
- 411:: a metal mesh

### [Best Mode]

Hereinafter, an embodiment of the present invention will be described with reference to the drawings.

A plasma generator 100 according to the present invention is used for a household appliance such as a refrigerator, a washing machine, a cleaner, a clothing dryer, an air conditioner, or an air cleaner, and serves to deodorize air in an indoor or outdoor of the household appliance and to sterilize floating bacteria or adhesive bacteria in the indoor or outdoor of the household appliance.

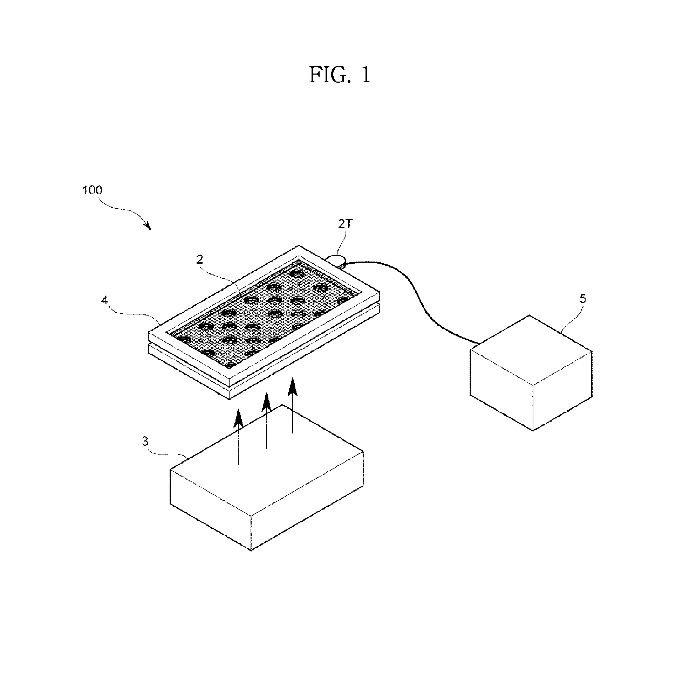

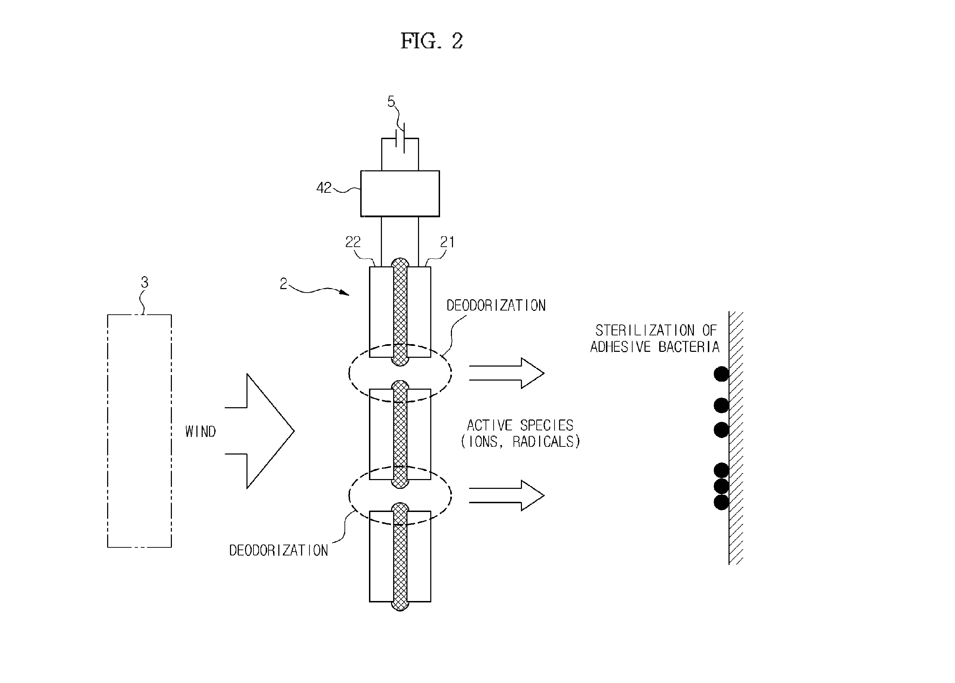

Specifically, as shown in FIGS. 1 and 2, the plasma generator 100 includes a plasma electrode portion 2 to generate active species such as ions and radicals using Micro Gap Plasma, a blower mechanism 3 which is provided outside the plasma electrode portion 2 to forcibly blow wind (an air stream) toward the plasma electrode portion 2, an explosion-proof mechanism 4 which is provided outside the plasma electrode portion 2 so that flame generated by the plasma electrode portion 2 is not spread to the outside, and a power source 5 to apply a high voltage to the electrode portion.

Hereinafter, the respective portions 2 to 5 will be described with reference to the drawings.

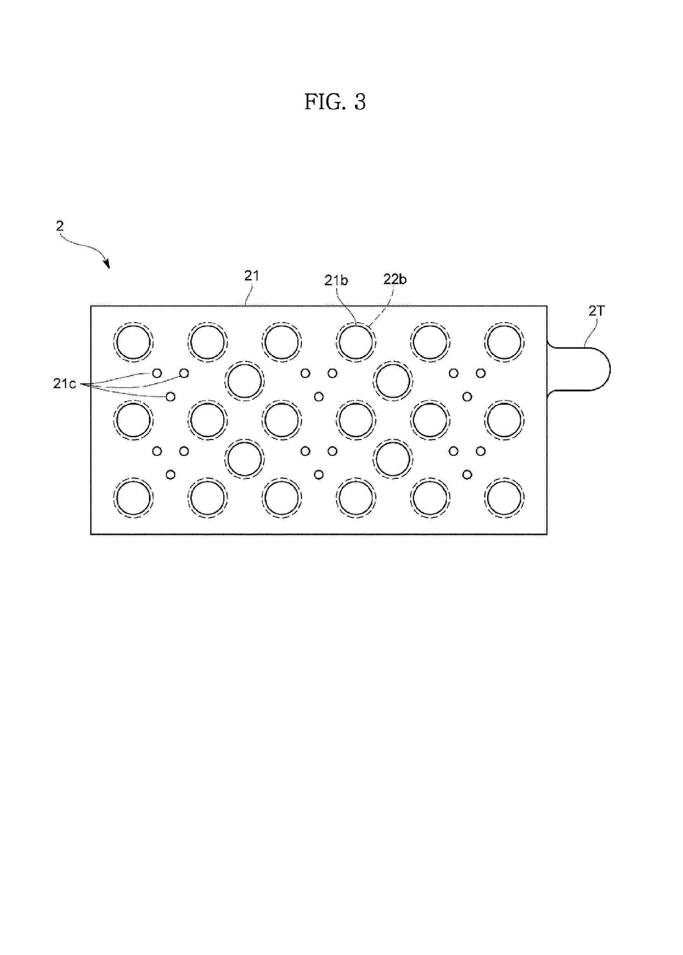

As shown in FIGS. 2 to 6, the plasma electrode portion 2 has a pair of electrodes 21 and 22 provided with dielectric films 21a and 22a on respective facing surfaces thereof, and serves to apply a predetermined voltage between the electrodes 21 and 22 and discharge plasma. In particular, as shown in FIG. 3, each of the electrodes 21 and 22 has a substantially rectangular shape in the plan view (when viewed from a face plate direction of the electrode 21 or 22), and is made of stainless steel such as SUS403, for example. An edge portion of the electrode 21 or 22 of the electrode portion 2 is formed with an applied terminal T to which a voltage is applied from the power source 5 (see FIG. 3). Here, a method of applying the voltage to plasma electrode portion 2 by the power source 5 is made by forming the voltage applied to each electrode 21 or 22 in a pulse shape, setting a peak value thereof within a range of 100 V to 5000 V, and setting a pulse width within a range of 0.1 µs to 300 µs.

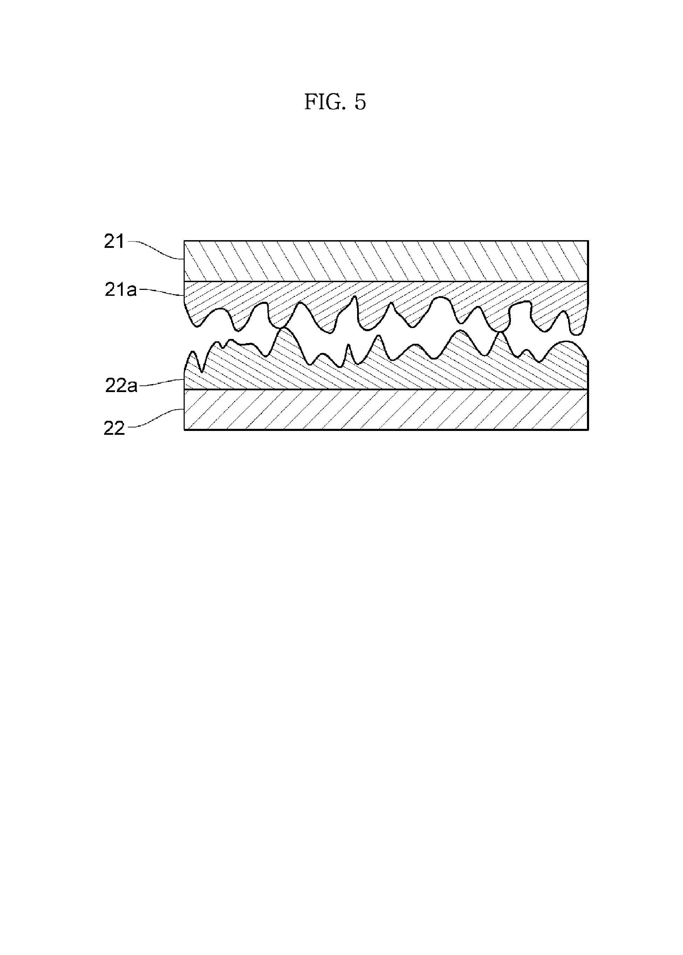

In addition, as shown in FIG. 5, the respective facing surfaces of the electrodes 21 and 22 are formed with the dielectric films 21a and 22a by application of dielectric such as barium titanate, for example. The dielectric films 21a and 22a have surface roughness (calculation mean roughness Ra in the embodiment) of 0.1 µm to 100 µm. These other surface roughness may also be defined using a maximum height Ry and ten point mean roughness Rz. A gap is defined between the facing surfaces by adjusting plane roughness of the dielectric films 21a and 22a to a value within the above range and just overlapping the respective electrodes 21 and 22, so that plasma is generated within the gap. Thus, a spacer to define a gap for plasma formation between the respective electrodes 21 and 22 is not required. In addition, the surface roughness of the dielectric films 21a and 22a is considered to be controlled by sputtering. In addition, aluminum oxide, titanium oxide, magnesium oxide, strontium titanate, silicon oxide, silver phosphate, lead zirconate titanate, silicon carbide, indium oxide, cadmium oxide, bismuth oxide, zinc oxide, iron oxide, carbon nanotube, or the like may also be used as the dielectric applied to the electrodes.

Furthermore, as shown in FIGS 3, 4, and 6, the electrodes 21 and 22 are respectively provided with fluid circulation holes 21b and 22b at corresponding positions corresponding of the respective electrodes 21 and 22 such that the respective electrodes 21 and 22 are configured to be penetrated as a whole by communication of the fluid circulation holes 21b and 22b. At the same time, at least a portion of an outline of each corresponding fluid circulation hole 21b or 22b is configured so as to be arranged at positions different from each other. In other words, a shape of the fluid circulation hole 21b formed in the electrode 21 of one side when viewed from the plane differs from a shape of the fluid circulation hole 22b formed in the electrode 21 of the other side when viewed from the plane.

Specifically, the fluid circulation holes 21b and 22b which are respectively formed at the corresponding positions of the respective electrodes 21 and 22 are a substantially circular shape when viewed from the plane (see FIG. 3). An opening size (opening diameter) of the fluid circulation hole 21b formed in the electrode 21 of one side is smaller (for example, the opening diameter is small by 10 µm or more) than an opening size (opening diameter) of the fluid circulation hole 22b formed in the electrode 22 of the other side.

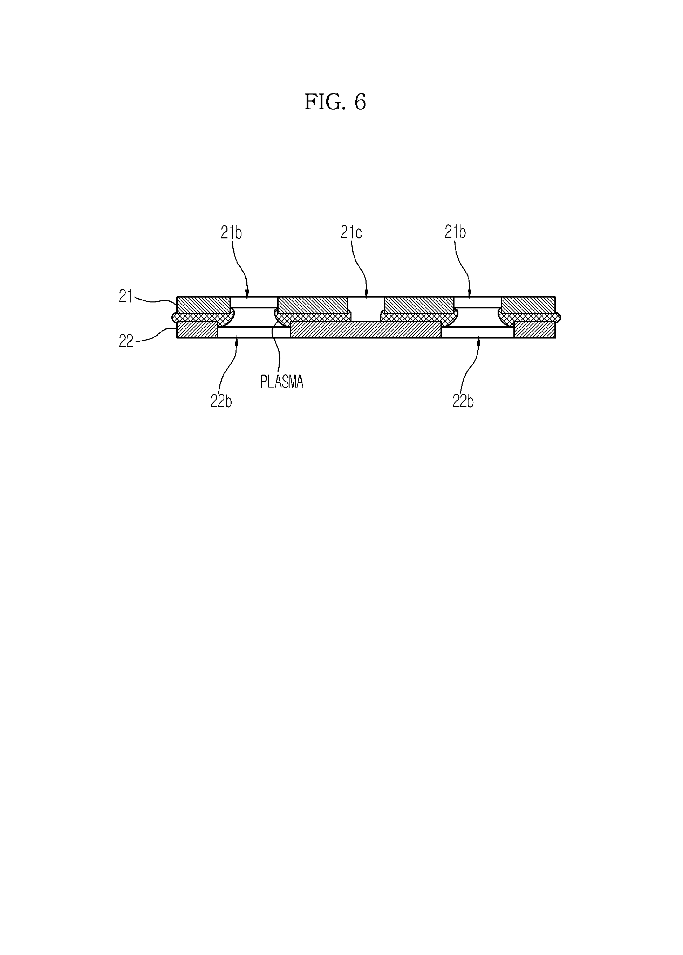

In addition, as shown in FIGS. 3 and 6, the fluid circulation hole 21b formed in the electrode 21 of one side and the fluid circulation hole 22b formed in the electrode 22 of the other side are formed in a concentric circular shape. In the present embodiment, a plurality of fluid circulation holes 21b formed in the electrode 21 of one side have the same shape as a whole, and a plurality of fluid circulation holes 22b formed in the electrode 22 of the other side also have the same shape as a whole. All of the fluid circulation holes 21b formed in the electrode 21 of one side are smaller than the fluid circulation holes 22b formed in the electrode 22 of the other side. Although shown as a substantially circular shape so that an effect is realized in the present embodiment, the opening portion is not limited as to being formed in a circular shape. For example, at least a portion of the outline of each corresponding fluid circulation hole when viewed from the plane may be configured so as to be arranged at the positions different from each other.

Furthermore, a total opening area of the fluid circulation holes 21b or 22b formed in each electrode 21 or 22 is within a range of 2% to 90% with respect to a total area of each electrode 21 or 22. Specifically, the total opening area of the fluid circulation holes 22b formed in the electrode 22 of the other side is set within a range of 2% to 90% with respect to the total are of the electrode 22. Moreover, the total opening area of the fluid circulation holes 21b formed in the electrode 21 of one side may also be set within a range of 2% to 90%.

However, the plasma electrode portion 2 in the present embodiment, as shown in FIGS. 3 and 6, is configured so that a through hole 21c is provided separately from the fluid circulation holes 21b and 22b in the electrode 21 of one side and the through hole 21c is blocked, at an opening of the facing surface thereof, by the electrode 22 of the other side. Hereinafter, a portion made from the fluid circulation hole 21b or 22b formed in each electrode 21 or 22 is referred to as a full opening portion, whereas a portion formed by the through hole 21c is referred to as a half opening portion.

The opening size of the through hole 21c is formed to be smaller than the opening size of the fluid circulation hole 21b by 10 µm or more. The through hole 21c is formed by replacing a portion of the fluid circulation holes 21b which regularly arranged, and the through hole 21c is provided around the fluid circulation hole 21b (see FIG. 3).

The blower mechanism 3 is disposed on the side of the other electrode 22 of the plasma electrode portion 2, and has a blowing fan which forcibly sends wind toward the fluid circulation holes (full opening portions) 21b and 22b formed in the plasma electrode portion 2. Specifically, the blower mechanism 3 allows a flow rate of the wind passing through the fluid circulation holes 21b and 22b to be within a range of 0.1 m/s to 10 m/s.

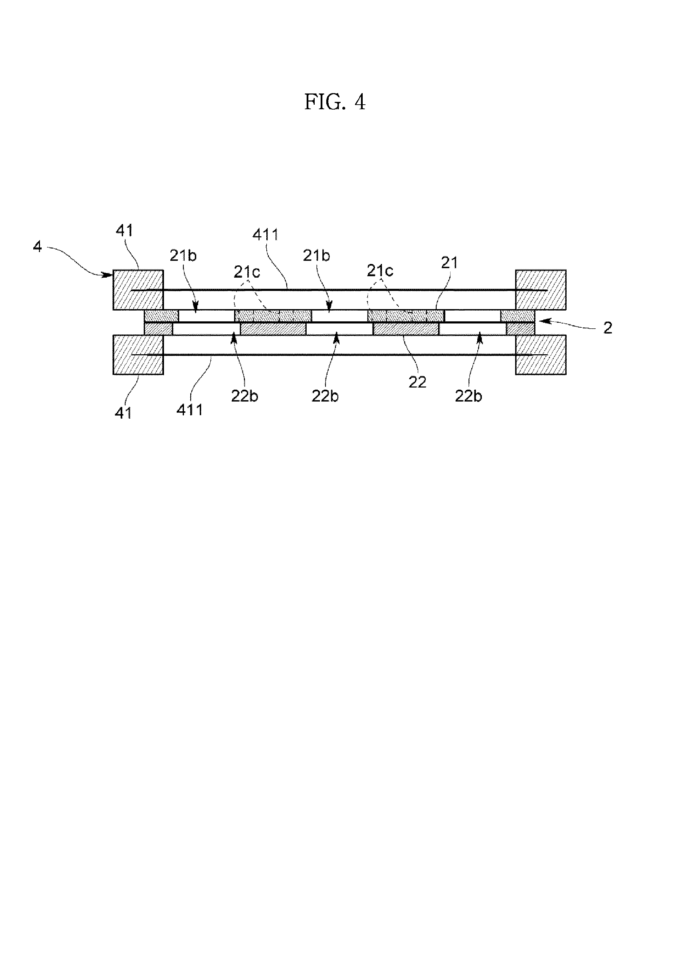

As shown in FIG. 4, the explosion-proof mechanism 4 has protective covers 41 disposed to the outer sides of the pair of electrodes 21 and 22, and is configured so that flame generated by plasma by introduction of inflammable gas into the fluid circulation holes 21b and 22b is not spread beyond the protective covers 41 to the outside. Specifically, the explosion-proof mechanism 4 has metal meshes 411 in which the protective covers 41 are disposed at the outer sides of the pair of electrodes 21 and 22. The wire diameter of each metal mesh 411 is within a range of 1.5 mm or less, the opening ratio of the metal mesh 411 is 30% or more.

The plasma generator 100 having such a configuration performs deodorization in the vicinity of the electrodes 21 and 22 by generating plasma in the gap between two opposite electrodes 21 and 22 and sending wind to the fluid circulation holes 21b and 22b, and performs sterilization of adhesive bacteria by releasing active species generated in the plasma to a closed space. Here, since products generated in the plasma are wholly transported downstream by the wind, there is a need to limit generation of ozone harmful to a human body. Therefore, it may be possible to suppress ozone generation and enable both deodorization and sterilization by optimizing parameters such as a structure of the full opening portion of each electrode 21 or 22, addition of the half opening portion, a shape of the opening portion, voltage control, and wind speed. In addition, even when the closed space is filled with inflammable gas, the explosion-proof mechanism 4 is provided so as to be safely operated, and optimization is performed so as not to reduce performance of deodorization and sterilization due to the explosion-proof mechanism 4.

Next, the following description will be given with respect to an experimental example using the plasma generator 100 of the present embodiment. The optimization of an electrode shape is executed by air ion measurement and ozone concentration measurement in order to perform both sterilization and deodorization of adhesive bacteria. Both measurement are carried out in a distance which may install a measuring instrument downstream than the plasma electrode portion 2 (in this case, an inlet port is installed at the position of 1 cm in the ozone concentration measurement and at the position of 10 cm in the ion number density measurement). The air ion measurement is a method which is indirect, but is conveniently measured. In the air ion measurement method, although an object to be measured is ions which particularly have a charge and a long life among the active species generated in the plasma, a correlation between the air ion number density and the density of the active species is used under conditions of generating constant plasma. That is, the ion number density being high means that the density of the active species which are effective in sterilization and deodorization is high. Meanwhile, since ozone which is a by-product of plasma has a very long life (a few ten minutes or more) compared to ions, there is no significant difference between concentration in the vicinity of plasma and concentration at a point away from the downstream. Nevertheless, in order to increase the absolute value of a measured value and catch a small change in generation amount of ozone, a sampling inlet port of the measuring instrument is installed downstream apart from the electrode 21 by 1 cm. In such a measuring system, when the ion number density is maximized based on the ozone concentration, this is directly connected to the optimization of an electrode shape.

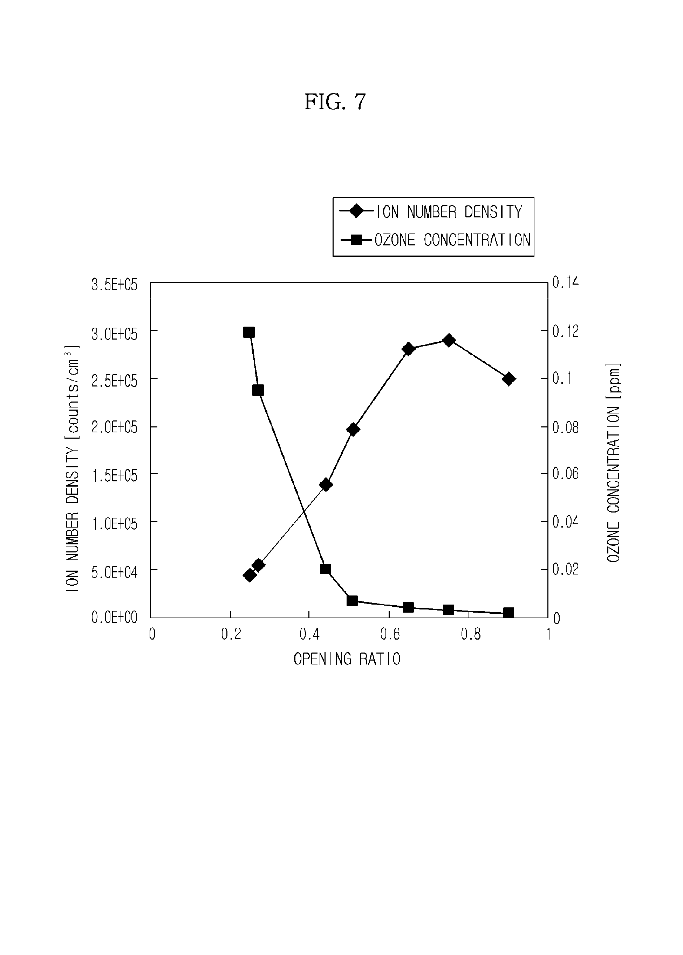

FIG. 7 shows a measurement result of ion number density of ozone concentration when the opening ratio of the fluid circulation hole 22b (fluid circulation hole 21b) is varied. The ion number density is increased together with an increase in opening ratio, whereas the ozone concentration is decreased. As can be seen from FIG. 7, a ratio (opening ratio) of the total opening area of the fluid circulation holes 22b formed in the electrode 22 to the total area of the electrode 22 is preferably set within a range of 40% to 90%, and more preferably a range of 40% to 80%.

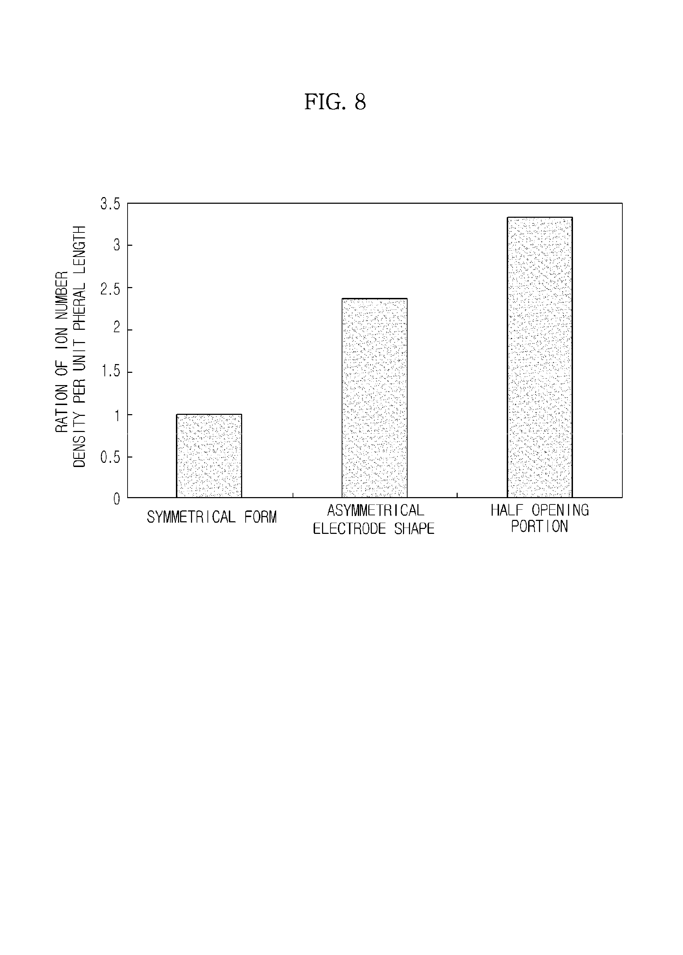

An asymmetrical structure of the above electrode and an increase in generation amount of ions by the half opening portion are confirmed as follows.
The three types of electrodes having the same opening ratio are prepared as follows:
1) an electrode which includes only a symmetrical full opening portion (the fluid circulation opening 21b and the fluid circulation opening 22b have the same shape) as a basis;
2) an electrode which changes the full opening portion into an asymmetrical structure (a configuration in the present embodiment); and
3) an electrode which includes a half opening portion in addition to the symmetrical full opening portion.
An applied voltage is adjusted so that the ozone concentration is constantly kept in the respective electrodes, and the ion number density generated by these conditions is measured by the above method. Subsequently, the total extension of the peripheral length of the opening portion on the electrode is obtained, and ion number density per unit peripheral length is calculated from the measured ion number density. The electrodes 1) and 2) are directly compared with each other and are changed into an asymmetrical form, thus an increased amount is obtained, and an increased amount by the half opening portion by subtracting the ion number density of 1) from the ion number density of 3). FIG. 8 shows a ratio of the generation amount of ions to the symmetrical full opening portion. As shown in FIG. 8, it is determined that the generation amount of ions is increased by two times or more by being changed into the asymmetrical form (full opening portion in the present embodiment), and the generation amount of ions from the half opening portion is increased by three times or more the symmetrical form. Furthermore, by promoting this structure of the electrode, and arrangement on the electrodes of asymmetrical full opening portion and the half opening portion, it may be possible to increase a generation amount of active species at maximum 100 times.

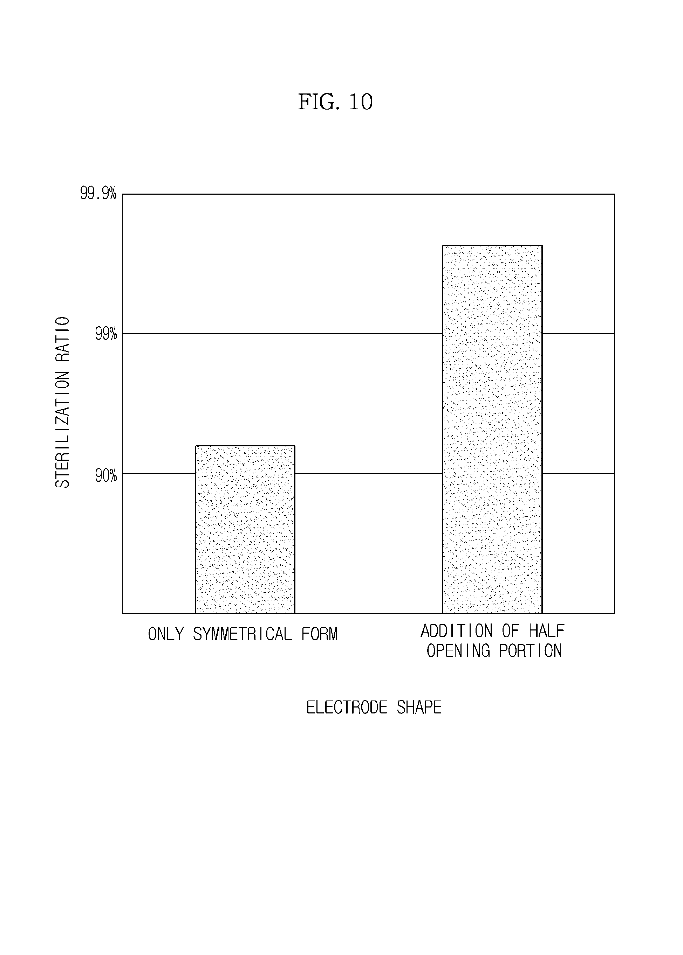

FIG. 10 shows a result which improves sterilization ability by changing the electrode structure and increasing the generation amount of active species. An object to be sterilized is colon bacteria, the active species are released for six hours with respect to a medium to which the colon bacteria are applied in 100 L container at room temperature, and then the bacteria are cultured on the medium and the number of the formed colons counts. As a result, the sterilization efficiency is increased more than one column by adding the half opening portion from the electrode of only the symmetrical opening portion. At the generation conditions of active species, approximately complete sterilization (99.9%) is predicted as being realized within eight hours. For example, this sterilization ability is sufficient to completely sterilize the adhesive bacteria in the storage during transportation of a refrigerator for one day.

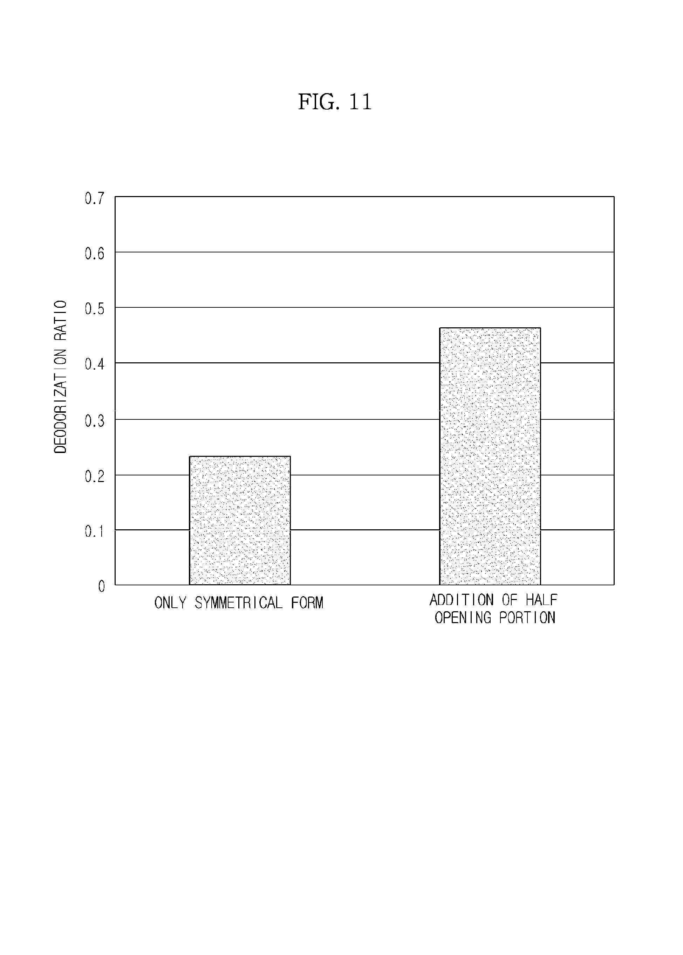

Similarly, FIG. 11 shows a change in deodorization ability by changing the electrode structure. The deodorization ability is obtained from the decomposition rate of odor when, at room temperature, 2 ppm of trimethylamine (TMA) as odor is injected into a 100 L capacity container made of resin and the plasma generator of the present embodiment is intermittently operated for two hours. It may be possible to increase deodorization efficiency by almost two times by adding the half opening portion.

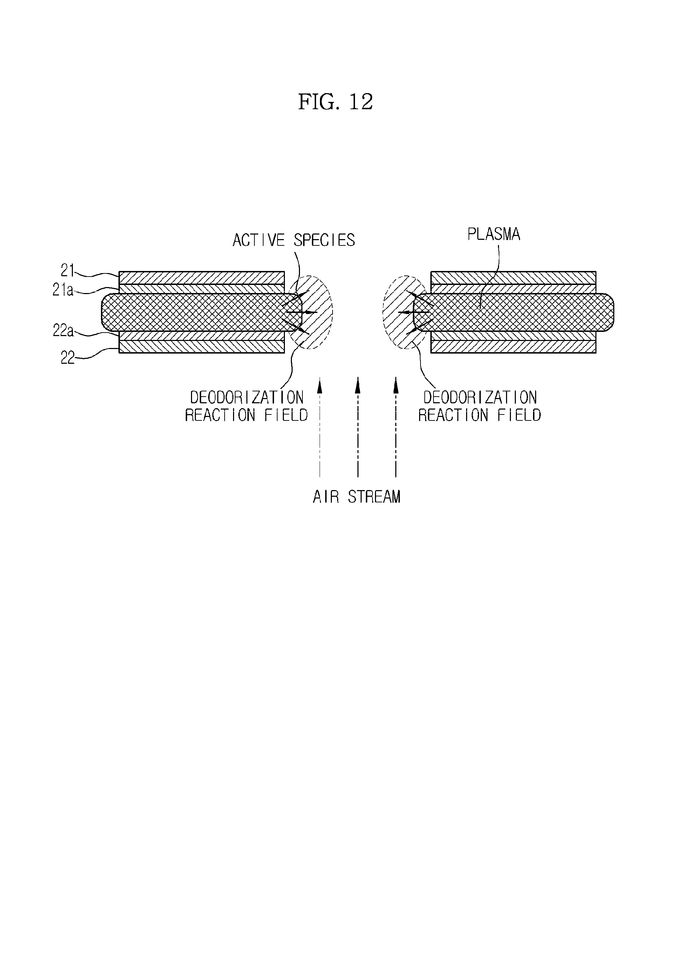

The deodorization reaction performed in the vicinity of the electrode is considered as follows. A difference between the concentration of the active species generated by plasma and the deodorization concentration transported by the air stream is considered. As shown in FIG. 12, since a portion of plasma generated in the gap defined by dielectrics on the electrode surface is spread up to the inside of the opening of the full opening portion, the generated active species mutually act with the air stream supplied from the blowing portion. The electronic density of plasma generated in a space interposed between the dielectrics is about 10¹⁵/cm³, the density of ions or radicals is equal. The molecular number density is 10¹³/cm³.

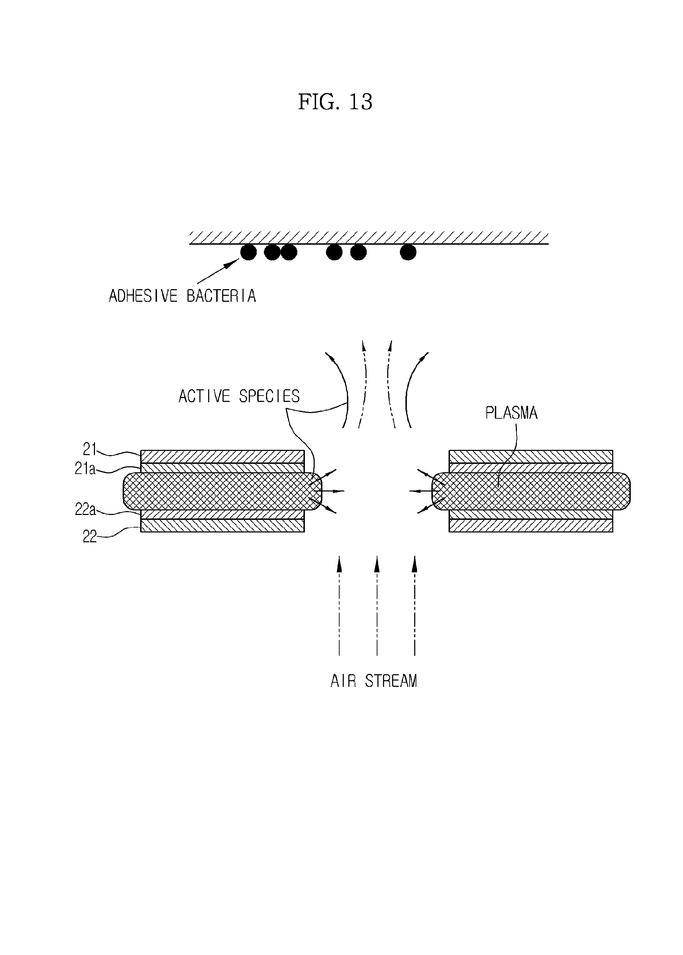

Next, although sterilization is performed on the surface spaced apart from the electrodes 21 and 22, the density difference of active species and adhesive bacteria determines sterilization efficiency. As shown in FIG. 13, the active species generated by the plasma and released downstream is returned to a safe molecule. The ion existing in the air is measured by an air ion measuring instrument, and is 10⁶/cm³ in the vicinity of the plasma. The adhesive bacteria are from several hundred to several thousand, namely, 10²/cm³ to 10³/cm³.

The deodorization reaction performed in the vicinity of the electrode is considered as follows. A difference between the concentration of the active species generated by plasma and the deodorization concentration transported by the air stream is considered. As shown in FIG. 12, since a portion of plasma generated in the gap defined by dielectrics on the electrode surface is spread up to the inside of the opening of the full opening portion, the generated active species mutually act with the air stream supplied from the blowing portion. The electronic density of plasma generated in a space interposed between the dielectrics is about 10¹⁵/cm³, the density of ions or radicals is equal.

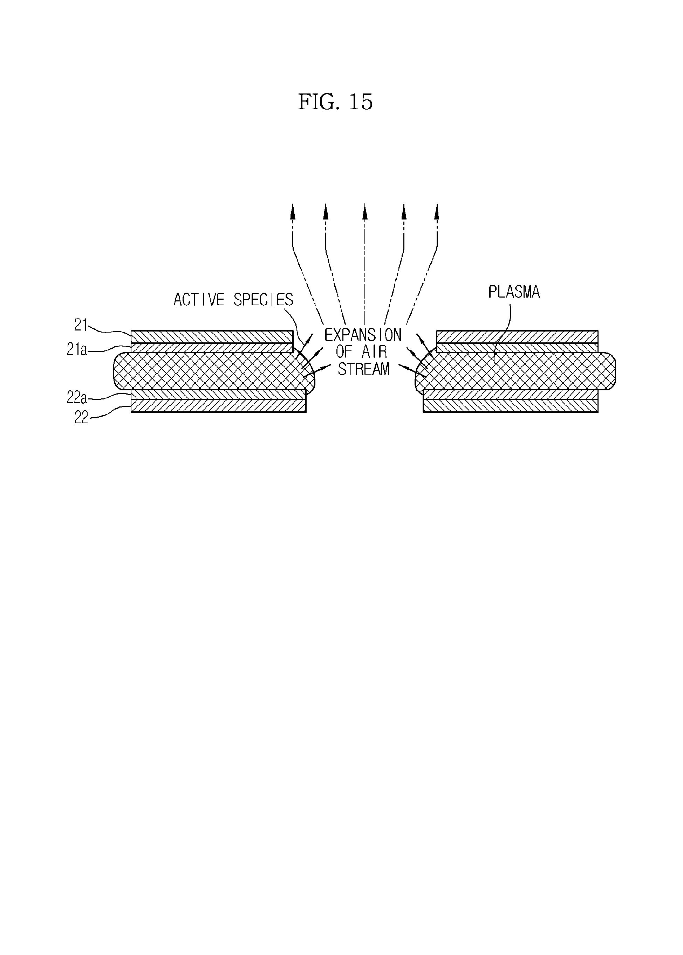

On the other hand, in a case where the size of the opening is increased (all of the fluid circulation holes 21b of the electrode 21 are formed to be smaller or larger than the plural fluid circulation holes 22b of the electrode 22), as shown in FIG. 15, it may be possible to increase sterilization efficiency by a negative pressure.

In addition, the pulse shape also includes a waveform in which the voltage rises along a saturation curve associated with the charging and discharging of a load and the voltage drops along an attenuation curve. Furthermore, in the detailed shape of the pulse wave, the pulse wave includes a symmetrical waveform in which a shape during the voltage rise and a shape during the voltage drop are equal to each other, and an asymmetrical waveform in which the respective shapes differ from each other. Actually, the generation of plasma results in the same effects that after the voltage becomes sufficiently high, the duration of the discharge is less than the half-width, and the pulse width becomes smaller.

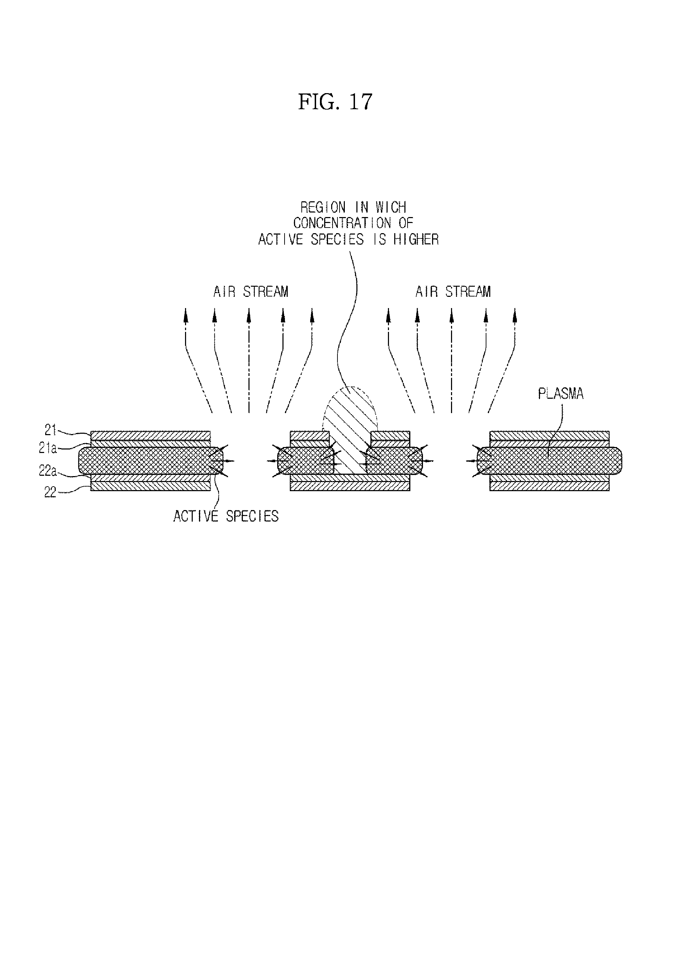

As shown in FIG. 17, when only the downstream side of the air stream is opened, the concentration of active species is increased in a region in which the air stream does not exist. By mutually acting with the region of the high concentration active species on the interface of the air stream, the active species are released, the ion number density is increased, and the sterilization efficiency is enhanced.

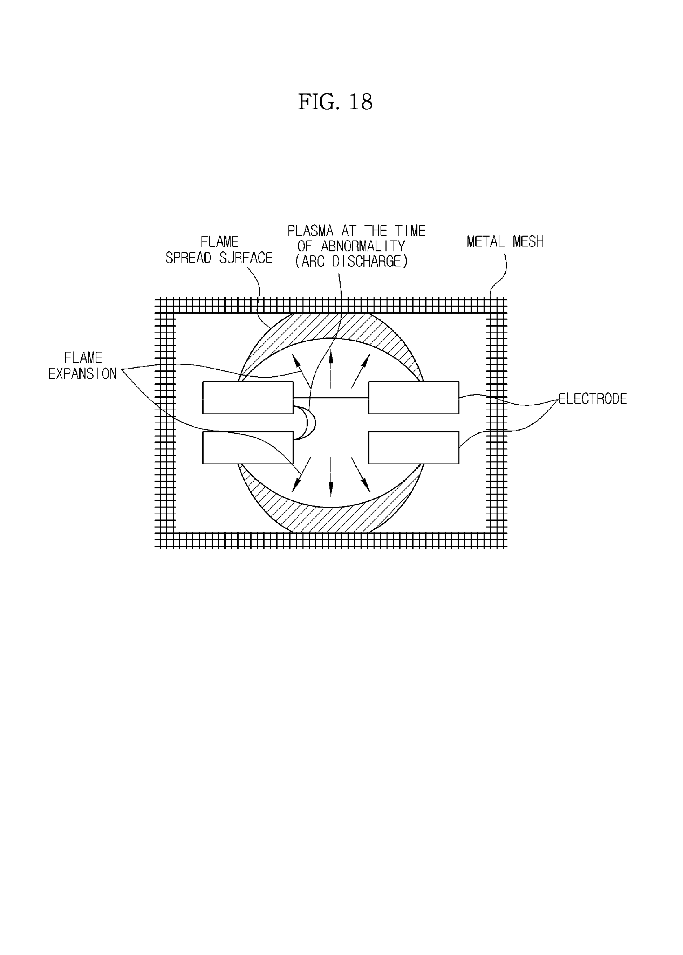

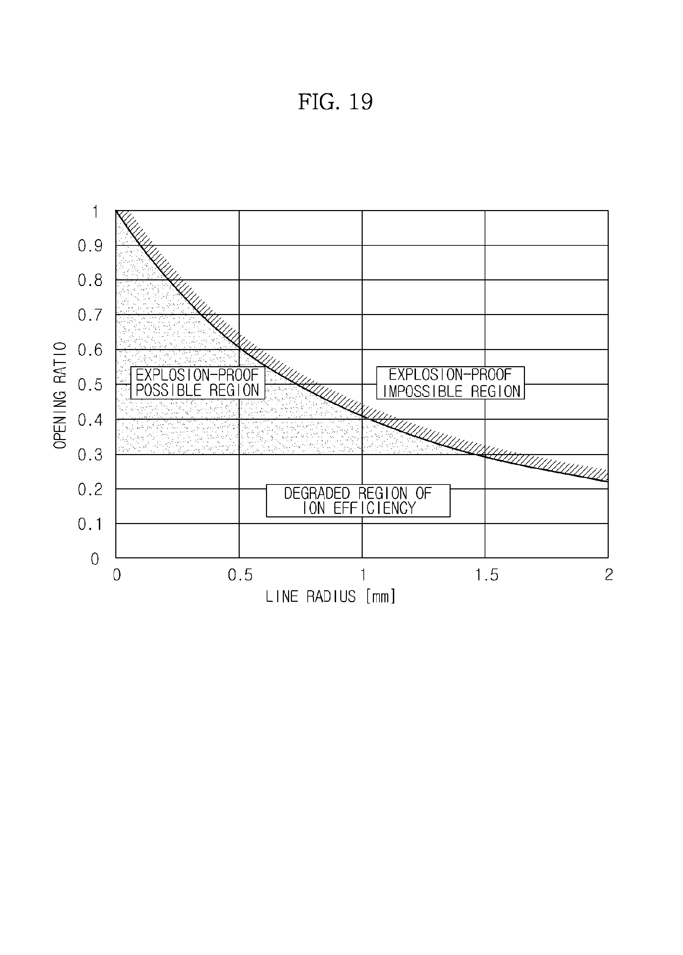

The explosion-proof mechanism 4 is required when the present device is installed in the refrigerator using inflammable refrigerant. As shown in FIG. 18, the metal mesh is arranged around the plasma electrode portion 2. Particularly, as shown in FIG. 19, it may be possible to be operated without deterioration of the generation amount of active species when the wire diameter of the metal mesh 411 is within a range of 1.5 mm or less, and the opening ratio is 30% or more. Thus, it may be possible to secure safety without deterioration of the deodorization ability and the sterilization ability.

### [an effect of the present embodiment]

In accordance with the plasma generator 100, since at least a portion of an outline of each corresponding fluid circulation hole 21b or 22b is arranged at positions different from each other, it may be possible to increase a contact area between fluid passing through the fluid circulation hole 21b or 22b and the plasma. Thus, it is possible to increase a generation amount of active species such as ions or radicals and to sufficiently realize a deodorization function by the active species and a function which releases the active species outside an apparatus to sterilize floating bacteria and adhesive bacteria.

### [other modification embodiment]

The present is not limited to the above embodiment. For example, although the plural fluid circulation holes 21b of the electrode 21 have the same shape and the plural fluid circulation holes 22b of the electrode 22 have the same shape in the embodiment, other shapes may also be formed.

In addition, although all of the fluid circulation holes 21b of the electrode 21 are formed to be smaller or larger than the plural fluid circulation holes 22b of the electrode 22 in the above embodiment, a portion of the fluid circulation holes 21b of the electrode 21 may be small by the fluid circulation holes 22b of the electrode 22 and other fluid circulation holes 21b of the electrode 21 may be formed to be larger than fluid circulation holes 22b of the electrode 22.

Furthermore, although the through hole is formed at either the electrode 21 of one side or the electrode 22 of the other side, the through hole (half opening portion) may be formed at both thereof.

Moreover, although the fluid circulation holes have the same cross-section shape, the fluid circulation hole formed in the electrode may have a tapered surface, a conical shape or bowl shape. That is, the fluid circulation hole may have a reduced diameter or an enlarged diameter as being advanced from one opening to the other opening.

The fluid circulation hole may have at least any one of a circular shape, an elliptical shape, a rectangular shape, a linear slit shape, a concentric circular slit shape, a waveform slit shape, a lunular shape, a comb shape, a honeycomb shape, and a star shape, when viewed from the face plate direction of the electrode.

In addition, the present invention is not limited to the above embodiment, and various modifications are possible without departing from the scope and spirit of the invention.

### [Industrial Applicability]

In accordance with the present invention, it may be possible to suppress a generation amount of ozone while increasing a generation amount of active species.
Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A plasma generator comprising:
a pair of electrodes, in a case where a predetermined voltage is applied between the electrodes to discharge plasma, fluid circulation holes being respectively provided at corresponding positions of each electrode and passing through the electrode, at least a portion of an outline of each corresponding fluid circulation hole being arranged at positions different from each other when viewed from a face plate direction of the electrode.

2. The plasma generator according to claim 1, wherein at least one side of the pair of electrodes is provided with a dielectric film.

3. The plasma generator according to claim 1, wherein a size of the fluid circulation hole formed in the electrode of one side of the pair of electrodes is formed to be smaller than a size of the fluid circulation hole formed in the electrode of the other side by 10 µm or more.

4. The plasma generator according to claim 1, wherein the fluid circulation hole formed in the electrode of one side and the fluid circulation hole formed in the electrode of the other side are arranged in a concentric circular shape.

5. The plasma generator according to claim 1, wherein the corresponding fluid circulation holes of the pair of electrodes are provided in plural number.

6. The plasma generator according to claim 5, wherein the fluid circulation hole has the same cross-section shape, or has a reduced diameter or an enlarged diameter as being advanced from one opening to the other opening.

7. The plasma generator according to claim 1, wherein the fluid circulation hole has at least any one of a circular shape, an elliptical shape, a rectangular shape, a linear slit shape, a concentric circular slit shape, a waveform slit shape, a lunular shape, a comb shape, a honeycomb shape, and a star shape, when viewed from the face plate direction of the electrode.

8. The plasma generator according to claim 1, wherein a total opening area of the fluid circulation holes formed in each electrode is within a range of 2% to 90% with respect to a total area of each electrode.

9. The plasma generator according to claim 1, wherein a through hole is provided separately from the fluid circulation holes in the electrode of one side and the through hole is blocked, at an opening of a facing surface thereof, by the electrode of the other side.

10. The plasma generator according to claim 9, wherein an opening size of the through hole is formed to be smaller than an opening size of the fluid circulation hole by 10 µm or more.

11. The plasma generator according to claim 1, wherein surface roughness of the dielectric film is 0.1 µm to 100 µm.

12. The plasma generator according to claim 1, further comprising a blower mechanism to forcibly blow wind toward the fluid circulation holes.

13. A plasma generator comprising:
a pair of electrodes, in a case where a predetermined voltage is applied between the electrodes to discharge plasma, fluid circulation holes being respectively provided at corresponding positions of each electrode and passing through the electrode, a through hole being provided separately from the fluid circulation holes in the electrode of one side and the through hole being blocked, at an opening of a facing surface thereof, by the electrode of the other side.

14. The plasma generator according to claim 1, wherein a voltage applied to each electrode is formed in a pulse shape, a peak value thereof is set within a range of 100 V to 5000 V, and a pulse width is set within a range of 0.1 µs to 300 µs.

15. The plasma generator according to claim 1, further comprising an explosion-proof mechanism, wherein the explosion-proof mechanism has protective covers disposed to the outer sides of the pair of electrodes, and is configured so that flame generated by plasma through introduction of inflammable gas into the fluid circulation holes is not spread beyond the protective covers to the outside.

16. The plasma generator according to claim 15, wherein the protective covers has metal meshes disposed at the outer sides of the pair of electrodes, a wire diameter of each metal mesh is within a range of 1.5 mm or less, and an opening ratio of the metal mesh is 30% or more.

17. A plasma generating method using a pair of electrodes, wherein fluid circulation holes are respectively provided at corresponding positions of each electrode and pass through the electrode, at least a portion of an outline of each corresponding fluid circulation hole is arranged at positions different from each other when viewed from a face plate direction of the electrode, so that a predetermined voltage is applied between the electrodes to discharge plasma.

18. The plasma generator according to claim 17, wherein at least one side of the pair of electrodes is provided with a dielectric film.

## Amended claims

### Amended claims under Art. 19.1 PCT

**1.** (amended) A plasma generator comprising:
a pair of electrodes, in a case where a predetermined voltage is applied between the electrodes to discharge plasma between facing surfaces of the pair of electrodes, fluid circulation holes being respectively provided at corresponding positions of each electrode and passing through the electrode, at least a portion of an outline of each corresponding fluid circulation hole being arranged at positions different from each other when viewed from a face plate direction of the electrode.

**2.** (amended) The plasma generator according to claim 1, wherein a total opening area of the fluid circulation holes formed in each electrode is within a range of 60% to 90% with respect to a total area of each electrode.

**3.** The plasma generator according to claim 1, wherein a size of the fluid circulation hole formed in the electrode of one side of the pair of electrodes is formed to be smaller than a size of the fluid circulation hole formed in the electrode of the other side by 10 µm or more.

**4.** The plasma generator according to claim 1, wherein the fluid circulation hole formed in the electrode of one side and the fluid circulation hole formed in the electrode of the other side are arranged in a concentric circular shape.

**5.** The plasma generator according to claim 1, wherein the corresponding fluid circulation holes of the pair of electrodes are provided in plural number.

**6.** The plasma generator according to claim 5, wherein the fluid circulation hole has the same cross-section shape, or has a reduced diameter or an enlarged diameter as being advanced from one opening to the other opening.

**7.** The plasma generator according to claim 1, wherein the fluid circulation hole has at least any one of a circular shape, an elliptical shape, a rectangular shape, a linear slit shape, a concentric circular slit shape, a waveform slit shape, a lunular shape, a comb shape, a honeycomb shape, and a star shape, when viewed from the face plate direction of the electrode.

**8.** (amended) The plasma generator according to claim 1, wherein at least one side of the pair of electrodes is provided with a dielectric film.

**9.** The plasma generator according to claim 1, wherein a through hole is provided separately from the fluid circulation holes in the electrode of one side and the through hole is blocked, at an opening of a facing surface thereof, by the electrode of the other side.

**10.** The plasma generator according to claim 9, wherein an opening size of the through hole is formed to be smaller than an opening size of the fluid circulation hole by 10 µm or more.

**11.** The plasma generator according to claim 1, wherein surface roughness of the dielectric film is 0.1 µm to 100 µm.

**12.** The plasma generator according to claim 1, further comprising a blower mechanism to forcibly blow wind toward the fluid circulation holes.

**13.** A plasma generator comprising:
a pair of electrodes, in a case where a predetermined voltage is applied between the electrodes to discharge plasma, fluid circulation holes being respectively provided at corresponding positions of each electrode and passing through the electrode, a through hole being provided separately from the fluid circulation holes in the electrode of one side and the through hole being blocked, at an opening of a facing surface thereof, by the electrode of the other side.

**14.** The plasma generator according to claim 1, wherein a voltage applied to each electrode is formed in a pulse shape, a peak value thereof is set within a range of 100 V to 5000 V, and a pulse width is set within a range of 0.1 µs to 300 µs.

**15.** The plasma generator according to claim 1, further comprising an explosion-proof mechanism, wherein the explosion-proof mechanism has protective covers disposed to the outer sides of the pair of electrodes, and is configured so that flame generated by plasma through introduction of inflammable gas into the fluid circulation holes is not spread beyond the protective covers to the outside.

**16.** The plasma generator according to claim 15, wherein the protective covers has metal meshes disposed at the outer sides of the pair of electrodes, a wire diameter of each metal mesh is within a range of 1.5 mm or less, and an opening ratio of the metal mesh is 30% or more.

**17.** A plasma generating method using a pair of electrodes, wherein fluid circulation holes are respectively provided at corresponding positions of each electrode and pass through the electrode, at least a portion of an outline of each corresponding fluid circulation hole is arranged at positions different from each other when viewed from a face plate direction of the electrode, so that a predetermined voltage is applied between the electrodes to discharge plasma.

**18.** The plasma generator according to claim 17, wherein at least one side of the pair of electrodes is provided with a dielectric film.
